# EUROPÄISCHE PATENTANMELDUNG

(11) **EP 2 695 655 A1**
(43) Veröffentlichungstag der Anmeldung: **12.02.2014**
(21) Anmeldenummer: 12179900.1
(22) Anmeldetag: 09.08.2012
(51) Int. Cl.: B01D 33/01, G01N 33/49, G01N 1/40, A61B 10/00, B01L 3/00

(54) **Mehrteilige Vorrichtung zur Gewinnung von Plasma aus Vollblut**

(71) Anmelder: F. Hoffmann-La Roche AG, 4070 Basel (CH); Roche Diagnostics GmbH, 68305 Mannheim (DE)
(72) Erfinder: Huemer, Herfried, 8330 Feldbach (AT)
(74) Vertreter: Babeluk, Michael

(57) **Zusammenfassung**

Die Erfindung betrifft eine mehrteilige Vorrichtung zur Gewinnung von Plasma aus Vollblut, mit einer Probenentnahmeeinheit (11; 12) zur Aufnahme von Vollblut, einer Filtereinheit (10) aus einem mehrlagigen Schichtfilter zur Abtrennung des Plasmas und einer Pumpeinrichtung, vorzugsweise einer Kolbenpumpe (20), zur Erzeugung von Unterdruck in der Filtereinheit (10). Erfindungsgemäß sind die Filtereinheit (10) und ein in Richtung Filtereinheit (10) spitz zulaufender Plasmasammelbehälter (17) in einer Filterkartusche (13) angeordnet, die nach der Plasmagewinnung auftrennbar ist, so dass die Spitze (9) des Plasmasammelbehälters (17) zur Probeneingabe in einen Analysator frei gestellt ist

## Beschreibung

Die Erfindung betrifft eine mehrteilige Vorrichtung zur Gewinnung von Plasma aus Vollblut, mit einer Probenentnahmeeinheit zur Aufnahme von Vollblut, einer Filtereinheit aus einem mehrlagigen Schichtfilter zur Abtrennung des Plasmas und einer Pumpeinrichtung, vorzugsweise einer Kolbenpumpe, zur Erzeugung von Unterdruck in der Filtereinheit. Weiters betrifft die Erfindung eine Filterkartusche mit einer mehrlagigen Filtereinheit zur Gewinnung von Plasma aus Vollblut.

Neben den vor allem in Labors verwendeten Zentrifugen zur Plasmaabtrennung sind bereits eine Reihe von Vorrichtungen bekannt geworden, bei welchen auch im POC (Point of Care)-Bereich kleinste Plasmamengen durch Abtrennung aus Vollblut mittels Filtern zur Verfügung gestellt werden.

Im einfachsten Fall kann die Plasmaabtrennung durch einen mehrschichtigen Teststreifen gemäß DE 40 15 589 A1 (BOEHRINGER MANNHEIM) erfolgen, welcher auf einer inerten Trägerfolie eine Transportschicht zum Transport der Probenflüssigkeit (Vollblut) von einem Aufgabebereich in einen Messbereich aufweist. Die Transportschicht kann beispielsweise aus einem Glasfaservlies bestehen, das im Aufgabebereich von einer Plasmaabtrennschicht bedeckt ist. Das Verfahren eignet sich allerdings nur für Analysatoren, die mit Teststreifen arbeiten.

Aus der EP 0 550 950 A2 (SANWA KAGAKU KENKYUSHO) ist ein Verfahren und eine Vorrichtung zur Separation von Blutserum und Plasma bekannt. In diesem Dokument werden unterschiedliche Ausführungsvarianten von Vorrichtungen zur Plasmagewinnung dargestellt, wobei beispielsweise in den Figuren 1 bis 4 Varianten offenbart sind, bei welchen eine Plasmaabtrennvorrichtung in eine Blutgewinnungseinrichtung integriert ist. Das Blut wird zunächst mittels Unterdruck in einen Sammelbehälter gesaugt, in welchem ein zweischichtiges Trennfilter angeordnet ist. Nach Abschluss der Blutentnahme wird der Sammelbehälter mit einem evakuierten Fluidbehälter verbunden, wobei das Plasma durch das Trennfilter abgetrennt und im Fluidbehälter gesammelt wird. Bei einer Ausführungsvariante gemäß Fig. 5 und Fig. 6 wird der für die Plasmaabtrennung erforderliche Unterdruck durch eine Kolbenspritze erzeugt. Weiters zeigt die Ausführungsvariante gemäß Figuren 9 und 10 eine Art Spritzenvorsatzfilter, welches ebenfalls zur Plasmagewinnung verwendet werden kann.

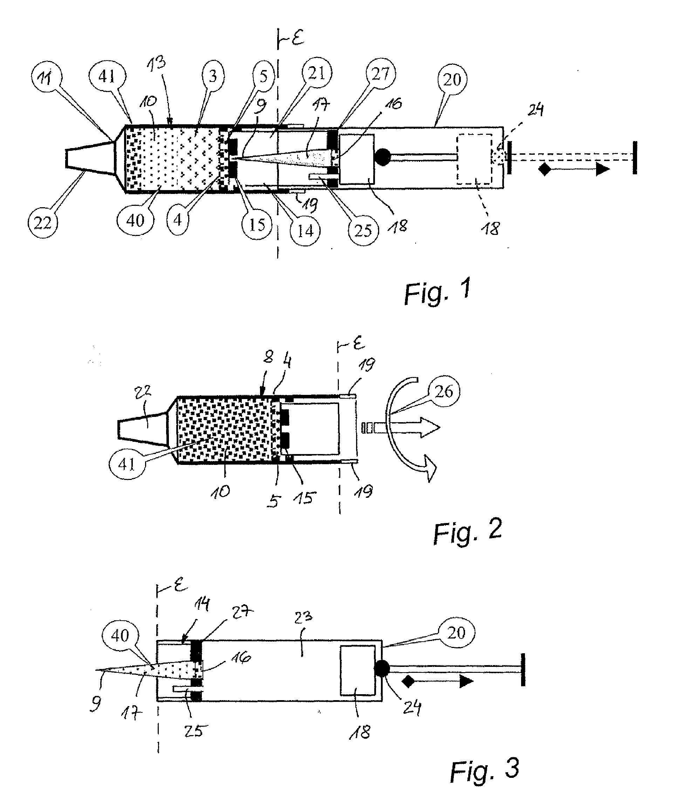

Aus der WO 96/24425 A1 (FIRST MEDICAL INC.), insbesondere deren Fig. 1 bis Fig. 3 und Fig. 8, ist eine Vorrichtung und ein Verfahren zur Plasmaabtrennung bekannt. Eine 'Blood Separation Device' genannte Einrichtung weist ein Filterelement, einen flexiblen Schlauch und an dessen Ende eine Nadel auf, welche in ein 'Blood Collection Device' eingeführt wird. Mit einer Antriebseinheit, die eine auf den flexiblen Schlauch einwirkende Schlauchpumpe aufweist, wird Vollblut aus dem 'Blood Collection Device' angesaugt und durch das Filterelement gepumpt, wobei Plasma separiert wird und an einer Plasma-Auslassöffnung der Filtereinheit für die weitere Verwendung zur Verfügung steht. Nachteilig sind die ungeregelten, relativ großen Druckwerte, die im Druckbetrieb vor der Filtereinheit auftreten, sowie der im Entnahmegefäß bei fortgesetzter Entnahme von Vollblut auftretende Unterdruck.

Aufgabe der Erfindung ist es, eine Vorrichtung zur Gewinnung von Plasma aus Vollblut vorzuschlagen, welche einfach und kostengünstig zu handhaben ist, wobei auch bei geringen Probenmengen und/oder hohen Hämatokritwerten für nachfolgende Anwendungsschritte brauchbare Plasmaproben gewonnen und auf einfache Weise der Eingabeeinrichtung eines Analysators zugeführt werden können.

Diese Aufgabe wird erfindungsgemäß dadurch gelöst, dass die Filtereinheit und ein in Richtung Filtereinheit spitz zulaufender Plasmasammelbehälter in einer Filterkartusche angeordnet sind, die nach der Plasmagewinnung auftrennbar ist, so dass die Spitze des Plasmasammelbehälters zur Probeneingabe in einen Analysator frei gestellt ist.

Bevorzugt kann die Filterkartusche durch Abziehen, Abdrehen oder Abschrauben eines Plasma-Applikators, der den spitz zulaufenden Plasmasammelbehälter enthält, von einem Filtergehäuse, das die Filtereinheit enthält, aufgetrennt werden. Der spitz zulaufende Plasmasammelbehälter kann nach der Plasmagewinnung und dem Abtrennen des Plasma-Applikators direkt mit dessen Spitze am Einfüllmund eines Analysators angedockt und die so gewonnene Plasma probe zur nachfolgenden Analytbestimmung in den Analysator eingesaugt werden.

Bevorzugt durchsetzt der Plasmasammelbehälter mit seiner Spitze eine Dichtung zur Filtereinheit und ist am gegenüberliegenden Ende durch eine Klemmdichtung gehalten, die eine Durchgangsöffnung zur Pumpeinrichtung, beispielsweise einer Kolbenpumpe, aufweist.

Die Dichtung zur Filtereinheit und die Klemmdichtung in der Filterkartusche begrenzen ein Totvolumen bzw. Ausgleichsvolumen, das über eine luftdurchlässige Verbindung, beispielsweise eine Ausgleichsöffnung oder eine poröse Membran, mit der Pumpeinrichtung verbunden ist. In vorteilhafter Weise erfolgt dadurch die Beaufschlagung der Filtereinheit nicht unkontrolliert direkt (abhängig von der Handhabung am Kolbenteil der Pumpeinrichtung), sondern langsam und gleichmäßig abfallend, wobei die Druckverhältnisse durch die geometrischen Abmessungen (beispielsweise das Verhältnis des Saugvolumens der Kolbenpumpe zum Ausgleichsvolumen in der Filterkartusche) der einzelnen Bauteile der Separationsvorrichtung und die Kenndaten des Filterelements eingestellt werden können.

Gegenstand der Erfindung ist weiters eine Filterkartusche mit einer mehrlagigen Filtereinheit zur Gewinnung von Plasma aus Vollblut, die im Inneren einen spitz zulaufenden Plasmasammelbehälter aufweist, dessen Spitze eine Dichtung zur Filtereinheit durchsetzt, wobei der Plasmasammelbehälter am gegenüberliegenden Ende durch eine Klemmdichtung in der Filterkartusche gehalten ist. Die Filterkartusche kann im Inneren einer Kolbenspritze oder einer Monovette angeordnet, integriert oder in diese eingesetzt sein.

Die Erfindung wird im Folgenden anhand von Zeichnungen näher erläutert. Es zeigen:
- Fig. 1: eine erste Ausführungsvariante (Kolbenspritze) der erfindungsgemäßen Vorrichtung zur Gewinnung von Plasma aus Vollblut in einer Schnittdarstellung;
- Fig. 2: den ersten Teil der Vorrichtung gemäß Fig. 1 nach der Plasmagewinnung;
- Fig. 3: den zweiten Teil der Vorrichtung gemäß Fig. 1 nach der Plasmagewinnung;

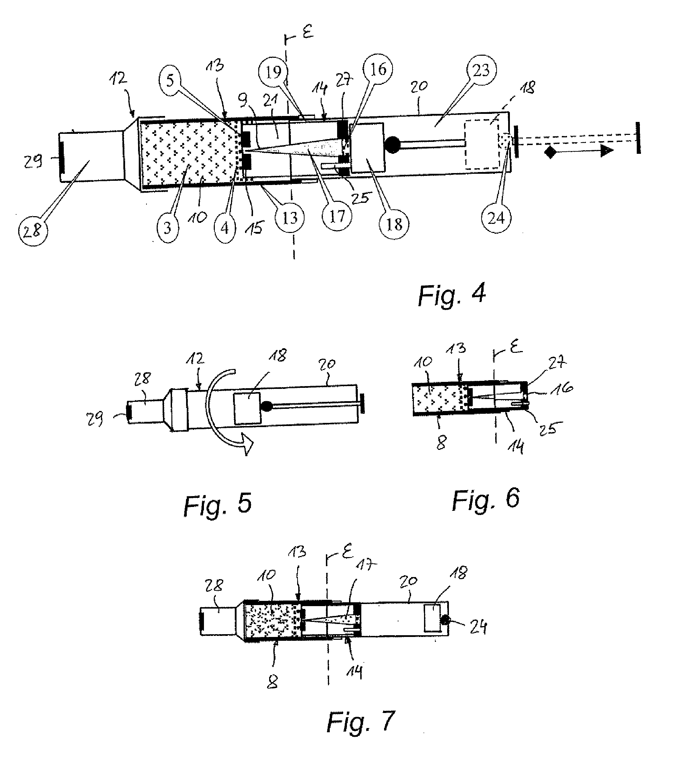

- Fig. 4: eine zweite Ausführungsvariante (Monovette) der erfindungsgemäßen Vorrichtung zur Gewinnung von Plasma aus Vollblut in einer Schnittdarstellung;
- Fig. 5: eine Monovette vor der Plasmagewinnung gemäß Fig. 4;
- Fig. 6: eine erfindungsgemäße Filterkartusche zur Gewinnung von Plasma aus Vollblut, einsetzbar in eine Monovette gemäß Fig. 5; sowie
- Fig. 7: die Filterkartusche gemäß Fig. 6 eingesetzt in die Monovette gemäß Fig. 5 in einer Schnittdarstellung.

Allen Ausführungsvarianten gemeinsam ist eine Filterkartusche 13, entweder als integraler Bestandteil der Probenentnahmeeinheit (siehe Kolbenspritze 11 gemäß Fig. 1 bis Fig. 3) oder als separates Bauteil, das vom Anwender in eine Probenentnahmeeinheit eingesetzt wird (siehe Monovette 12 gemäß Fig. 4 bis Fig. 7). Die Filterkartusche 13 weist eine Filtereinheit 10 mit einem mehrlagigen Schichtfilter, sowie einen in Richtung Filtereinheit 10 spitz zulaufenden Plasmasammelbehälter 17 (Plasma-Tip) auf. Die Filterkartusche 13 ist nach der Plasmagewinnung in einer Ebene ε in einen Plasma-Applikator 14, der den Plasmasammelbehälter 17 enthält und ein Filtergehäuse 8, das die Filtereinheit 10 aufweist, teilbar.

Die in den Fig. 1 bis Fig. 3 dargestellte erste Ausführungsvariante zeigt eine in eine Kolbenspritze 11 integrierte Filterkartusche 13, bei der am Filtergehäuse 8 ein Luer-Konus 22 angeformt ist, auf den zur Abnahme von Vollblut eine Nadel aufgesteckt werden kann. Im Gehäuse der Kolbenspritze 11, in deren Kolbengehäuse 20 ein per Hand mittels Kolbenstange von einer Ausgangsstellung in eine strichliert dargestellte Endstellung verschiebbarer Kolben 18 angeordnet ist, sind eine Filtereinheit 10 und ein in Richtung der Filtereinheit 10 spitz zulaufender Plasmasammelbehälter 17 als wesentliche Teile der Filterkartusche angeordnet. Das Kolbengehäuse 20 mit dem verschiebbaren Kolben 18 dient als Pumpeinrichtung zur Erzeugung des für die Plasmaabtrennung nötigen Unterdrucks.

Bei Betätigung der Vorrichtung wird durch Zurückziehen des Kolbens 18 die Vollblutprobe 41 in die Filtereinheit 10 eingesaugt, wobei sich eine Plasmafront oder Plasmafraktion 40 ausbildet, die durch das mehrlagige Schichtfilter, bestehend aus einem Tiefenfilter 3, einer feinporigeren Stoppmembran 4 zur vollständigen Antrennung fester Blutbestandteile (vor allem roter Blutkörperchen, RBKs) und einem Lateralgitter 5, bis zur Spitze 9 des Plasmasammelbehälters 17 wandert.

Der Plasmasammelbehälter 17 mit seiner Spitze 9 durchsetzt eine Dichtung 15 zur Filtereinheit 10 und wird am gegenüberliegenden, offenen Ende durch eine Klemmdichtung 27 gehalten und abgedichtet, die eine Durchgangsöffnung zur Pumpeinrichtung (Kolbenteil 20) aufweist. In der Durchgangsöffnung zur Pumpeinrichtung ist ein hydrophobes, luftdurchlässiges Element 16 (Liquid-Stop) angeordnet. Dieses verhindert, dass das gewonnene Plasma an dieser der Spitze 9 gegenüberliegenden Durchgangsöffnung ausfließen kann.

Die Dichtung 15 zur Filtereinheit 10 und die Klemmdichtung 27 zum Kolbengehäuse 20 begrenzen in der Filterkartusche 13 ein Ausgleichsvolumen 21, das über eine luftdurchlässige Verbindung, beispielsweise eine Ausgleichsöffnung 25 in der Klemmdichtung 27 oder eine poröse Membran, mit der Pumpeinrichtung der Kolbenspritze 11 verbunden ist.

Bei der Plasmagewinnung mit einer ersten Variante der Vorrichtung gemäß Fig. 1 bis Fig. 3 kann beispielsweise wie folgt vorgegangen werden:
● Entnahme der Kolbenspritze 11 mit integrierter Filterkartusche 13 aus einer sterilen Verpackung.
● Aufstecken einer Nadel auf den Luer-Konus 22.
● Einstechen in ein ausgewähltes Blutgefäß.
● Ansaugen einer Blutprobe durch Zurückziehen des Kolbens 18 bis zum Anschlag.
● Einrasten der Kolbenstange in eine Raststellung 24.
   *(Option: Abbrechen der Kolbenstange, um eine Umkehrung der internen Strömungsrichtung bzw. Unterdruckschwankungen und damit verbundene Verunreinigung des gewonnenen Plasmas bei hohem Hämatokrit und*/*oder geringer Probenmenge zu verhindern.)*

Das Tiefenfilter 3 der Filtereinheit 10 kann z.B. aus bindemittelfreien Glasfasern (typ. FV-2, Fa. Whatman bzw. DE 40 15 589 A1 bzw. EP 0 239 002 Böhringer-Mannheim) mit Rückhaltebereich von 0,5 µm bis 10 µm, besser 1 µm bis 5 µm, vorzugsweise typ. < 3 µm, aufgebaut sein. Die roten Blutkörperchen (RBKs) lagern sich an den feinen Glasfasern des Tiefenfilters 3 an, ohne die Flussrate übermäßig zu beeinflussen bzw. zu zerplatzen.
● Abhängig von Querschnitt der Filtereinheit 10 und vom Hämatokrit bildet sich eine "Plasmafront" oder "Plasmafraktion" 40 aus, die ungehindert durch die Stoppmembran 4 treten kann. Dabei werden durch die Stoppmembran 4 die restlichen RBKs herausgefiltert, welche durch den Tiefenfilter nicht zurückgehalten wurden. Hierzu weist die Stoppmembran 4 in Vergleich zum Tiefenfilter 3 eine deutlich geringere Porengröße auf, beispielsweise Poren mit einem Durchmesser von weniger als 400 nm, bevorzugt mit einem Durchmesser von weniger als 200 nm. Durch die Kombination eines Tiefenfilters 3, welcher aufgrund seiner Porengröße bereits den ganz überwiegenden Teil der Blutzellen zurückhält, aber die Plasmafraktion weitgehend ungestört durchfließen lässt, mit einer nachgeschalteten Stoppmembran 4, welche aufgrund ihrer kleineren Porengröße auch die verbleibenden Blutzellen zuverlässig zurückhält, aber aufgrund ihrer begrenzten Porenzahl ohne den vorgeschalteten Tiefenfilter 4 sehr rasch verstopfen würde, kann eine zuverlässige Abtrennung der Blutzellen ohne rasches Verstopfen des Filters erzielt werden, wodurch die Gewinnung eines ausreichenden Volumens einer Plasmaprobe ermöglicht wird.
● Der eingestellte Unterdruck in der Filtereinheit 10 von max. 500 mbar, besser 300 mbar, ideal 100 mbar bis 150 mbar, bestimmt mit den geometrischen Verhältnissen (Verhältnis des Ausgleichsvolumens 21 zum Saugvolumen 23 des Kolbengehäuses 20) auch die Flussrate und somit die Scherkräfte, die in der Stoppmembran 4 der Filtereinheit 10 speziell auf die RBKs einwirken. Ein Zerplatzen (Hämolyse) kann durch eine Optimierung des Ausgleichsvolumens 21 wirksam verhindert werden.
● Das Lateralgitter 5 der Filtereinheit 10 ermöglicht ein Sammeln und Absaugen des Plasmas nach der Stoppmembran 4 in Richtung Plasmasammelbehälter bzw. Plasmasammelbehälter 17, indem ein "Abdichten" durch die Stoppmembran 4 wirksam verhindert wird. Das Lateralgitter 5 bildet durch seine Gitterstruktur einerseits eine nicht durchgängige Auflage für die Stoppmembran 4, so dass Plasma auf der ausgangsseitigen Seite der Stoppmembran 4 ausfließen kann. Die Gitterstruktur bewirkt durch die Bildung von Kanälen weiterhin, dass das flächig aus der Stoppmembran 4 hindurchtretende Plasma im Bereich des Plasmasammelbehälters 17 zusammenfließen kann.
   *(Diese Funktion des Lateralgitters 5 kann alternativ auch durch entsprechende Strukturierung, beispielsweise durch Prägen, der der Stoppmembran zugewanden Seite der Dichtung 15 Bodens der Dichtung 15 bzw. durch deren ausreichende Rauhigkeit sichergestellt werden).*
● Die Plasmagewinnung wird insbesondere auch beendet durch:
   - ein Verschließen der Stoppmembran 4 durch partikuläre Blutbestandteile, oder
   - insbesondere bei Hämatokritwerten < ca. 40%, durch ein hydrophobes, luftdurchlässiges Element 16 (Liquid-Stopp) am Ende des Plasmasammelbehälters 17 bzw. in der Durchgangsöffnung der Klemmdichtung 27, welches eine weitere Plasmagewinnung begrenzt, sobald das durch das hydrophobe, luftdurchlässige Element 16 begrenzte Lumen des Plasmasammelbehälters 17 komplett mit dem gewonnenen Plasma gefüllt ist.
● Durch eine optische Kontrolle mittels Markierungen auf dem Plasmasammelbehälter 17 kann optional überprüft werden, ob die gewünschte Plasmamenge gewonnen werden konnte.
● Abschrauben oder Abdrehen (siehe Pfeil 26 in Fig. 2) des Filtergehäuses 8 der Kolbenspritze 11 vom Kolbenteil 20 und dadurch Auftrennung der Filterkartusche 13 in einer Ebene ε in einen ersten Teil, der die Filtereinheit 10 enthält und einen Plasma-Applikator-Teil 14, der den spitz zulaufenden Plasmasammelbehälter 17 enthält, so dass die Spitze 9 des Plasmasammelbehälters 17 frei gestellt ist (Fig. 3).
   Die inhärent höhere Haftreibung der Klemmdichtung 27 im Vergleich zur Dichtung 15 des Plasmasammelbehälters 17 mit kleinerer Dichtfläche sorgt in der dargestellten Ausführungsform für ein sicheres Abdocken der Spitze 9 des Plasmasammelbehälters 17, bevor durch weiteres Drehen bzw. Abziehen des Gehäuses Belüftungskanäle 19 in der Gehäusewand freigegeben werden, welche einen raschen Druckausgleich zwischen Saugvolumen 23 im Kolbengehäuse 20 und Ausgleichsvolumen 21 entweder über die zumindest teilweise poröse Klemmdichtung 27 und/oder die Ausgleichsöffnung 25 ermöglichen.
   Ein vorzeitiges Abdocken stellt weiters sicher, dass es beim Abtrennvorgang nicht zu unvorhersehbaren Komplikationen und Verunreinigungen an der Spitze 9 des Plasmasammelbehälters 17 kommen kann.
   Der Liquid-Stopp 16 am Ende des Plasmasammelbehälters 17 in der Klemmdichtung 27 verhindert auch ein Fraktionieren der Plasmaprobe im Bereich der Tip-Spitze im Falle eines verzögerten Druckausgleiches zwischen Saugvolumen 23 und Ausgleichsvolumen 21.
   *(Alternativ kann die Spitze 9 des Plasmasammelbehälters 17 auch als Luer-Kegel gestaltet sein).*
● Andocken des mit dem gewonnenen Plasma zumindest teilweise befüllten Plasmasammelbehälters 17 (samt Kolbengehäuse 20 als Griffstück) an den Einfüllmund eines nicht weiter dargestellten Analysators.
● Einbringen der Plasmaprobe in den Analysator durch analysatorseitiges Einsaugen aus dem am Einfüllmund des Analysators angedockten Plasmasammelbehälter 17. Die Ausgleichsöffnung 25 bzw. alternativ die porösen Bereiche der Klemmdichtung 27 erlauben hierbei ein vollständiges Entleeren des Plasmasammelbehälters 17.
● analytische Bestimmung der Inhaltsstoffe, beispielsweise der Hämoglobinwerte, der erfindungsgemäß gewonnenen Plasmaprobe im Analysator

Bei der Plasmagewinnung mit einer zweiten Variante der Vorrichtung gemäß Fig. 4 bis Fig. 7 kann beispielsweise wie folgt vorgegangen werden:
● Entnahme einer Monovette 12 gemäß Fig. 5 aus einer sterilen Verpackung.
● Abschrauben der Adapterkappe 28 mit der Durchstechmembran 29 vom Kolbengehäuse 20.
● Einbau der Filterkartusche 13 zwischen Adapterkappe 28 und Kolbengehäuse 20 gemäß Fig. 4
● Herstellen eines Unterdrucks in der Monovette 12 durch:
   - Zurückziehen des Kolbens 18 bis zum Anschlag
   - Einrasten der Kolbenstange in eine strichliert dargestellte Raststellung 24
   - *(Option: Abbrechen der Kolbenstange, um eine Umkehrung der internen Strömungsrichtung zu verhindern)*
● Andocken der Durchstechmembran 29 der Adapterkappe 28 an eine Punktiernadel, z.B. Butterfly.
● Einsaugen der Blutprobe durch den in der Monovette 12 herrschenden Unterdruck.

Das Tiefenfilter 3 der Filtereinheit 10 kann z.B. aus bindemittelfreien Glasfasern (typ. FV-2, Fa. Whatman bzw. DE 40 15 589 A1 bzw. EP 0 239 002 Böhringer-Mannheim) mit Rückhaltebereich von 0,5 µm bis 10 µm, besser 1 µm bis 5 µm, vorzugsweise typ. < 3µm aufgebaut sein. Die roten Blutkörperchen (RBKs) lagern sich dadurch an den feinen Glasfasern des Tiefenfilters 3 an, ohne die Flussrate übermäßig zu beeinflussen bzw. zu zerplatzen.
● Abhängig von Querschnitt der Filtereinheit 10 und vom Hämatokrit bildet sich eine "Plasmafront" oder "Plasmafraktion" 40 aus, die ungehindert durch die Stoppmembran 4 treten kann. Dabei werden die restlichen RBKs herausgefiltert (Fig. 4), welche durch den Tiefenfilter nicht zurückgehalten wurden. Hierzu weist die Stoppmembran 4 in Vergleich zum Tiefenfilter 3 eine deutlich geringere Porengröße auf, beispielsweise Poren mit einem Durchmesser von weniger als 400 nm, bevorzugt mit einem Durchmesser von weniger als 200 nm. Durch die Kombination eines Tiefenfilters 3, welcher aufgrund seiner Porengröße bereits den ganz überwiegenden Teil der Blutzellen zurückhält, aber die Plasmafraktion weitgehend ungestört durchfließen lässt, mit einer nachgeschalteten Stoppmembran 4, welche aufgrund ihrer kleineren Porengröße auch die verbleibenden Blutzellen zuverlässig zurückhält, aber aufgrund ihrer begrenzten Porenzahl ohne den vorgeschalteten Tiefenfilter 4 sehr rasch verstopfen würde, kann eine zuverlässige Abtrennung der Blutzellen ohne rasches Verstopfen des Filters erzielt werden, wodurch die Gewinnung eines ausreichenden Volumens einer Plasmaprobe ermöglicht wird.
● Der eingestellte Unterdruck in der Filtereinheit 10 von max. 500 mbar, besser 300 mbar, ideal 100 mbar bis 150 mbar, bestimmt mit den geometrischen Verhältnissen (Verhältnis des Ausgleichsvolumens 21 zum Saugvolumen 23 des Kolbengehäuses 20) auch die Flussrate und somit die Scherkräfte, die in der Stoppmembran 4 der Filtereinheit 10 speziell auf die RBKs einwirken. Ein Zerplatzen (Hämolyse) kann durch Optimierung des Ausgleichsvolumens 21 wirksam verhindert werden.
● Das Lateralgitter 5 der Filtereinheit 10 ermöglicht ein Sammeln und Absaugen des Plasmas nach der Stoppmembran 4 in Richtung Plasmasammelbehälter 17, indem ein "Abdichten" durch die Stoppmembran 4 wirksam verhindert wird. (siehe auch Variante Kolbenspritze)
   *(Diese Funktion des Lateralgitters 5 kann alternativ auch durch entsprechende Strukturierung, beispielsweise durch Prägen, der der Stoppmembran zugewanden Seite der Dichtung 15 Bodens der Dichtung 15 bzw. durch deren ausreichende Rauhigkeit sichergestellt werden).*
● Die Plasmagewinnung wird insbesondere auch beendet durch:
   - ein Verschließen der Stoppmembran 4 durch partikuläre Blutbestandteile, oder
   - insbesondere bei Hämatokritwerten < ca. 40%, durch ein hydrophobes, luftdurchlässiges Element 16 (Liquid-Stopp) am Ende des Plasmasammelbehälters 17 bzw. in der Durchgangsöffnung der Klemmdichtung 27, welches eine weitere Plasmagewinnung begrenzt, sobald das durch das hydrophobe, luftdurchlässige Element 16 begrenzte Lumen des Plasmasammelbehälters 17 komplett mit dem gewonnenen Plasma gefüllt ist.
● Durch eine optische Kontrolle mittels Markierungen auf dem Plasmasammelbehälter 17, kann auch hier optional überprüft werden, ob die gewünschte Plasmamenge gewonnen werden konnte.
● Abschrauben oder Abdrehen des vorderen Teils der Monovette 12 vom Kolbenteil 20 und dadurch Auftrennung der dazwischen eingebrachten Filterkartusche 13 in ein Filtergehäuse 8, das die Filtereinheit 10 enthält und einen Plasma-Applikator 14, der den spitz zulaufenden Plasmasammelbehälter 17 enthält, in einer Ebene ε, so dass die Spitze 9 des Plasmasammelbehälters 17 frei gestellt ist (Fig. 7). Zur besseren Handhabung bleibt der Plasma-Applikator 14 mit dem Kolbengehäuse 20 verbunden. Nach der Abtrennung entspricht die Vorrichtung jener in Fig. 3.

Die weiteren Schritte entsprechen jenen bei der oben beschriebenen ersten Ausführungsvariante der erfindungsgemäßen Vorrichtung.

## Patentansprüche

1. Mehrteilige Vorrichtung zur Gewinnung von Plasma aus Vollblut, mit einer Probenentnahmeeinheit (11; 12) zur Aufnahme von Vollblut, einer Filtereinheit (10) aus einem mehrlagigen Schichtfilter zur Abtrennung des Plasmas und einer Pumpeinrichtung, vorzugsweise einer Kolbenpumpe (20), zur Erzeugung von Unterdruck in der Filtereinheit (10), **dadurch gekennzeichnet, dass** die Filtereinheit (10) und ein in Richtung Filtereinheit (10) spitz zulaufender Plasmasammelbehälter (17) in einer Filterkartusche (13) angeordnet sind, die nach der Plasmagewinnung auftrennbar ist, so dass die Spitze (9) des Plasmasammelbehälters (17) zur Probeneingabe in einen Analysator frei gestellt ist.

2. Vorrichtung nach Anspruch 1, **dadurch gekennzeichnet, dass** die Filterkartusche (13) durch Abziehen, Abdrehen oder Abschrauben eines Plasma-Applikators (14), der den spitz zulaufenden Plasmasammelbehälter (17) enthält, von einem Filtergehäuse (8), das die Filtereinheit (10) enthält, auftrennbar ist.

3. Vorrichtung nach Anspruch 1 oder 2, **dadurch gekennzeichnet, dass** der Plasmasammelbehälter (17) mit seiner Spitze (9) eine Dichtung (15) zur Filtereinheit (10) durchsetzt und am gegenüberliegenden Ende durch eine Klemmdichtung (27) gehalten ist, die eine Durchgangsöffnung zur Pumpeinrichtung aufweist.

4. Vorrichtung nach Anspruch 3, **dadurch gekennzeichnet, dass** die Dichtung (15) zur Filtereinheit (10) und die Klemmdichtung (27) in der Filterkartusche (13) ein Ausgleichsvolumen (21) begrenzen, das über eine luftdurchlässige Verbindung, beispielsweise eine Ausgleichsöffnung (25) oder eine poröse Membran, mit der Pumpeinrichtung verbunden ist.

5. Vorrichtung nach einem der Ansprüche 2 bis 4, **dadurch gekennzeichnet, dass** in der Durchgangsöffnung zur Pumpeinrichtung ein hydrophobes, luftdurchlässiges Element (16) angeordnet ist.

6. Vorrichtung nach einem der Ansprüche 1 bis 5, **dadurch gekennzeichnet, dass** das mehrlagige Schichtfilter der Filtereinheit (10) aus einem Tiefenfilter (3), einer Stoppmembran (4) und einem Lateralgitter (5) besteht.

7. Vorrichtung nach einem der Ansprüche 1 bis 6, **dadurch gekennzeichnet, dass** die Filterkartusche (13) im Inneren einer Kolbenspritze (11) angeordnet, integriert oder in diese einsetzbar ist.

8. Vorrichtung nach einem der Ansprüche 1 bis 6, **dadurch gekennzeichnet, dass** die Filterkartusche (13) im Inneren einer Monovette (12) angeordnet, integriert oder in diese einsetzbar ist.

9. Vorrichtung nach Anspruch 7 oder 8, **dadurch gekennzeichnet, dass** der Kolben (18) der Kolbenspritze (11) oder der Monovette (12) in seiner Ausgangsstellung vor der Plasmagewinnung an der Klemmdichtung (27) der Filterkartusche (13) anliegt und am Ende der Plasmagewinnung in einer Raststellung (24) einrastet.

10. Filterkartusche (13) mit einer mehrlagigen Filtereinheit (10) zur Gewinnung von Plasma aus Vollblut, **dadurch gekennzeichnet, dass** die Filterkartusche (13) im Inneren einen spitz zulaufenden Plasmasammelbehälter (17) aufweist, dessen Spitze (9) eine Dichtung (15) zur Filtereinheit (10) durchsetzt, wobei der Plasmasammelbehälter (17) am gegenüberliegenden Ende durch eine Klemmdichtung (27) in der Filterkartusche (13) gehalten ist.

11. Filterkartusche (13) nach Anspruch 10, **dadurch gekennzeichnet, dass** die Dichtung (15) zur Filtereinheit (10) und die Klemmdichtung (27) in der Filterkartusche (13) ein Ausgleichsvolumen (21) begrenzen und die Klemmdichtung (27) eine luftdurchlässige Ausgleichsöffnung (25) oder eine poröse Membran aufweist.

12. Filterkartusche (13) nach Anspruch 10 oder 11, **dadurch gekennzeichnet, dass** die Filterkartusche (13) durch Abziehen, Abdrehen oder Abschrauben eines Plasma-Applikators (14), der den spitz zulaufenden Plasmasammelbehälter (17) enthält, von einem Filtergehäuse (8), das die Filtereinheit (10) enthält, auftrennbar ist, so dass die Spitze (9) des Plasmasammelbehälters (17) zur Probeneingabe in einen Analysator frei gestellt ist.
